# EUROPEAN PATENT APPLICATION

(11) **EP 3 508 517 A1**
(43) Date of publication of application: **10.07.2019**
(21) Application number: 17846701.5
(22) Date of filing: 01.09.2017
(51) Int. Cl.: C08J 5/00, B29C 43/02, C07K 14/435, C08H 1/00, C08L 89/00

(54) **MOLDED BODY AND METHOD FOR PRODUCING MOLDED BODY**

(30) Priority: 02.09.2016 JP 2016171915
(71) Applicant: Spiber Inc., Yamagata 997-0052 (JP); Tekunohama Co., Ltd., Toyota-shi, Aichi 470-0441 (JP)
(72) Inventor: HIRAI Shinji, Muroran-shi Hokkaido 050-8585 (JP); UBUKATA Yukimasa, Muroran-shi Hokkaido 050-8585 (JP); ABE Ayumi, Tsuruoka-shi Yamagata 997-0052 (JP); KOTAKA Koichi, Tsuruoka-shi Yamagata 997-0052 (JP); NAKAYAMA Yuki, Toyota-shi Aichi 470-0441 (JP); NOBA Junichi, Toyota-shi Aichi 470-0441 (JP)
(74) Representative: Handley, Matthew Edward
(86) International application number: PCT/JP2017/031559
(87) International publication number: WO 2018/043698

(57) **Abstract**

The present invention provides a method for producing a molded article including a step 1 of adding water to a composition including a structural protein, a step 2 of molding a water-containing composition obtained in the step 1, and a step 3 of drying the molded composition obtained in the step 2 to obtain the molded article.

### Sequence Listing

SEQUENCE LISTING

<110>  Spiber Inc.
       TEKUNOHAMA CO., LTD
       MURORAN INSTITUTE OF TECHNOLOGY

<120>  MOLDED MATERIAL AND METHOD FOR MANUFACTURING THE SAME

<130>  FP17-0531-00

<160>  16

<170>  PatentIn version 3.5

<210>  1
<211>  597
<212>  PRT
<213>  Artificial Sequence

<220>
<223>  Met-PRT313

<400>  1

Met Gly Pro Gly Gly Gln Gly Pro Tyr Gly Pro Gly Ala Ser Ala Ala
1               5                   10                  15

Ala Ala Ala Gly Gly Asn Gly Pro Gly Ser Gly Gln Gln Gly Pro Gly
            20                  25                  30

Gly Ser Ala Ala Ala Ala Ala Gly Gly Tyr Gly Pro Gly Gly Gln Gly
        35                  40                  45

Pro Gly Gln Gln Gly Pro Gly Ser Ser Ala Ala Ala Ala Ala Gly Pro
    50                  55                  60

Gly Gly Tyr Gly Pro Gly Gly Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gly Tyr Gly Pro Gly Gly Gln Gly Pro Gly Gln Gln
            100                 105                 110

Gly Pro Gly Ser Ser Ala Ala Ala Ala Ala Gly Gly Tyr Gly Ser Gly
        115                 120                 125

Pro Gly Gln Gln Gly Pro Tyr Gly Ser Ala Ala Ala Ala Ala Gly Pro
    130                 135                 140

Gly Ser Gly Gly Tyr Gly Gln Gly Pro Tyr Gly Pro Gly Ala Ser Ala
145                 150                 155                 160

Ala Ala Ala Ala Gly Pro Gly Gly Tyr Gly Pro Gly Gly Gln Gly Pro
                165                 170                 175

Ser Ala Ser Ala Ala Ala Ala Ala Gly Ser Gly Gln Gln Gly Pro Gly
            180                 185                 190

Gly Tyr Gly Pro Tyr Ala Ser Ala Ala Ala Ala Ala Gly Gly Tyr Gly
        195                 200                 205

Ser Gly Pro Gly Gln Gln Gly Pro Tyr Gly Pro Gly Gly Ser Ala Ala
    210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gly Gln Gly Pro Tyr Gly
                245                 250                 255

Pro Gly Ser Ser Ala Ala Ala Ala Ala Gly Gly Tyr Gly Tyr Gly Pro
            260                 265                 270

Gly Gly Gln Gly Pro Tyr Gly Pro Gly Ala Ser Ala Ala Ala Ala Ala
        275                 280                 285

Gly Gly Asn Gly Pro Gly Ser Gly Gly Tyr Gly Pro Gly Gln Gln Gly
    290                 295                 300

Pro Gly Gly Ser Ala Ala Ala Ala Ala Gly Pro Gly Gly Gln Gly Pro
305                 310                 315                 320

Tyr Gly Pro Gly Ala Ser Ala Ala Ala Ala Ala Gly Gly Tyr Gly Pro
                325                 330                 335

Gly Gly Gln Gly Pro Gly Gly Tyr Gly Pro Gly Ser Ser Ala Ala Ala
            340                 345                 350

Ala Ala Gly Pro Gly Gly Gln Gly Pro Tyr Gly Pro Gly Ser Ser Ala
        355                 360                 365

Ala Ala Ala Ala Gly Gly Tyr Gly Pro Gly Gln Gln Gly Pro Tyr Gly
    370                 375                 380

Pro Gly Gly Ser Ala Ala Ala Ala Ala Gly Gly Tyr Gln Gln Gly Pro
385                 390                 395                 400

Gly Gly Gln Gly Pro Tyr Gly Pro Gly Ala Ser Ala Ala Ala Ala Ala
                405                 410                 415

Gly Pro Gly Gly Gln Gly Pro Tyr Gly Pro Gly Ala Ser Ala Ala Ala
                420                 425                 430

Ala Ala Gly Pro Gly Gly Tyr Gly Pro Gly Gly Gln Gly Pro Ser Ala
            435                 440                 445

Ser Ala Ala Ala Ala Ala Gly Gly Tyr Gly Ser Gly Pro Gly Gly Tyr
    450                 455                 460

Gly Pro Tyr Gly Pro Gly Gly Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gly Tyr Gly Pro Gly Gln Gln Gly Pro Tyr Gly Pro
            500                 505                 510

Gly Gly Ser Ala Ala Ala Ala Ala Gly Pro Gly Ser Gly Gly Tyr Gly
        515                 520                 525

Pro Gly Ala Ser Ala Ala Ala Ala Ala Gly Gly Asn Gly Pro Gly Ser
    530                 535                 540

Gly Gly Tyr Gly Pro Gly Gln Gln Gly Pro Gly Gly Ser Ala Ala Ala
545                 550                 555                 560

Ala Ala Gly Gly Tyr Gln Gln Gly Pro Gly Gly Gln Gly Pro Tyr Gly
                565                 570                 575

Pro Gly Ala Ser Ala Ala Ala Ala Ala Gly Pro Gly Ser Gly Gln Gln
            580                 585                 590

Gly Pro Gly Ala Ser
        595

<210>  2
<211>  590
<212>  PRT
<213>  Artificial Sequence

<220>
<223>  Met-PRT399

<400>  2

Met Gly Pro Gly Gly Gln Gly Pro Tyr Gly Pro Gly Ala Ser Ala Ala
1               5                   10                  15

Ala Ala Ala Gly Gly Asn Gly Pro Gly Ser Gly Gln Gln Gly Pro Gly
            20                  25                  30

Gly Ser Gly Gly Tyr Gly Pro Gly Gly Gln Gly Pro Gly Gln Gln Gly
        35                  40                  45

Pro Gly Ser Ser Ala Ala Ala Ala Ala Gly Pro Gly Gly Tyr Gly Pro
    50                  55                  60

Gly Gly Gln Gly Pro Ser Ala Ser Ala Ala Ala Ala Ala Gly Pro Gly
65                  70                  75                  80

Ser Gly Gln Gln Gly Pro Gly Ala Ser Gly Gly Tyr Gly Pro Gly Gly
                85                  90                  95

Gln Gly Pro Gly Gln Gln Gly Pro Gly Ser Ser Ala Ala Ala Ala Ala
            100                 105                 110

Gly Gly Tyr Gly Ser Gly Pro Gly Gln Gln Gly Pro Tyr Gly Ser Ala
        115                 120                 125

Ala Ala Ala Ala Gly Pro Gly Ser Gly Gly Tyr Gly Gln Gly Pro Tyr
    130                 135                 140

Gly Pro Gly Ala Ser Gly Pro Gly Gly Tyr Gly Pro Gly Gly Gln Gly
145                 150                 155                 160

Pro Ser Ala Ser Ala Ala Ala Ala Ala Gly Ser Gly Gln Gln Gly Pro
                165                 170                 175

Gly Gly Tyr Gly Pro Tyr Ala Ser Ala Ala Ala Ala Ala Gly Gly Tyr
            180                 185                 190

Gly Ser Gly Pro Gly Gln Gln Gly Pro Tyr Gly Pro Gly Gly Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gly Gln Gly Pro Tyr Gly Pro Gly Ser Ser
225                 230                 235                 240

Ala Ala Ala Ala Ala Gly Gly Tyr Gly Tyr Gly Pro Gly Gly Gln Gly
                245                 250                 255

Pro Tyr Gly Pro Gly Ala Ser Gly Gly Asn Gly Pro Gly Ser Gly Gly
            260                 265                 270

Tyr Gly Pro Gly Gln Gln Gly Pro Gly Gly Ser Ala Ala Ala Ala Ala
        275                 280                 285

Gly Pro Gly Gly Gln Gly Pro Tyr Gly Pro Gly Ala Ser Ala Ala Ala
    290                 295                 300

Ala Ala Gly Gly Tyr Gly Pro Gly Gly Gln Gly Pro Gly Gly Tyr Gly
305                 310                 315                 320

Pro Gly Ser Ser Gly Pro Gly Gly Gln Gly Pro Tyr Gly Pro Gly Ser
                325                 330                 335

Ser Ala Ala Ala Ala Ala Gly Gly Tyr Gly Pro Gly Gln Gln Gly Pro
            340                 345                 350

Tyr Gly Pro Gly Gly Ser Ala Ala Ala Ala Ala Gly Gly Tyr Gln Gln
        355                 360                 365

Gly Pro Gly Gly Gln Gly Pro Tyr Gly Pro Gly Ala Ser Gly Pro Gly
    370                 375                 380

Gly Gln Gly Pro Tyr Gly Pro Gly Ala Ser Ala Ala Ala Ala Ala Gly
385                 390                 395                 400

Pro Gly Gly Tyr Gly Pro Gly Gly Gln Gly Pro Ser Ala Ser Ala Ala
                405                 410                 415

Ala Ala Ala Gly Gly Tyr Gly Ser Gly Pro Gly Gly Tyr Gly Pro Tyr
            420                 425                 430

Gly Pro Gly Gly Ser Gly Pro Gly Ser Gly Gln Gln Gly Gln Gly Pro
        435                 440                 445

Tyr Gly Pro Gly Ala Ser Ala Ala Ala Ala Ala Gly Gly Tyr Gly Pro
    450                 455                 460

Gly Gln Gln Gly Pro Tyr Gly Pro Gly Gly Ser Ala Ala Ala Ala Ala
465                 470                 475                 480

Gly Pro Gly Ser Gly Gly Tyr Gly Pro Gly Ala Ser Gly Gly Asn Gly
                485                 490                 495

Pro Gly Ser Gly Gly Tyr Gly Pro Gly Gln Gln Gly Pro Gly Gly Ser
            500                 505                 510

Ala Ala Ala Ala Ala Gly Gly Tyr Gln Gln Gly Pro Gly Gly Gln Gly

515                 520                 525

Pro Tyr Gly Pro Gly Ala Ser Ala Ala Ala Ala Ala Gly Gly Tyr Gly
    530                 535                 540

Ser Gly Pro Gly Gln Gln Gly Pro Tyr Gly Pro Gly Gly Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  3
<211>  597
<212>  PRT
<213>  Artificial Sequence

<220>
<223>  Met-PRT380

<400>  3

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Ala Ala Ala Ala Ala Gly Gln Tyr Gly Pro Gly Gln Gln Gly
        35                  40                  45

Pro Gly Gln Gln Gly Pro Gly Ser Ser Ala Ala Ala Ala Ala Gly Pro
    50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gln Tyr Gly Pro Gly Gln Gln Gly Pro Gly Gln Gln
            100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
    130                 135                 140

Gly Ser Gly Gln Tyr Gly Gln Gly Pro Tyr Gly Pro Gly Ala Ser Ala
145                 150                 155                 160

Ala Ala Ala Ala Gly Pro Gly Gln Tyr Gly Pro Gly Gln Gln Gly Pro
                165                 170                 175

Ser Ala Ser Ala Ala Ala Ala Ala Gly Ser Gly Gln Gln Gly Pro Gly
            180                 185                 190

Gln Tyr Gly Pro Tyr Ala Ser Ala Ala Ala Ala Ala Gly Gln Tyr Gly
        195                 200                 205

Ser Gly Pro Gly Gln Gln Gly Pro Tyr Gly Pro Gly Gln Ser Ala Ala
    210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gln Gln Gly Pro Tyr Gly
                245                 250                 255

Pro Gly Ser Ser Ala Ala Ala Ala Ala Gly Gln Tyr Gly Tyr Gly Pro
            260                 265                 270

Gly Gln Gln Gly Pro Tyr Gly Pro Gly Ala Ser Ala Ala Ala Ala Ala
        275                 280                 285

Gly Gln Asn Gly Pro Gly Ser Gly Gln Tyr Gly Pro Gly Gln Gln Gly
    290                 295                 300

Pro Gly Gln Ser Ala Ala Ala Ala Ala Gly Pro Gly Gln Gln Gly Pro
305                 310                 315                 320

Tyr Gly Pro Gly Ala Ser Ala Ala Ala Ala Ala Gly Gln Tyr Gly Pro
                325                 330                 335

Gly Gln Gln Gly Pro Gly Gln Tyr Gly Pro Gly Ser Ser Ala Ala Ala
            340                 345                 350

Ala Ala Gly Pro Gly Gln Gln Gly Pro Tyr Gly Pro Gly Ser Ser Ala
        355                 360                 365

Ala Ala Ala Ala Gly Gln Tyr Gly Pro Gly Gln Gln Gly Pro Tyr Gly
    370                 375                 380

Pro Gly Gln Ser Ala Ala Ala Ala Ala Gly Gln Tyr Gln Gln Gly Pro

385                 390                 395                 400

Gly Gln Gln Gly Pro Tyr Gly Pro Gly Ala Ser Ala Ala Ala Ala Ala
                405                 410                 415

Gly Pro Gly Gln Gln Gly Pro Tyr Gly Pro Gly Ala Ser Ala Ala Ala
            420                 425                 430

Ala Ala Gly Pro Gly Gln Tyr Gly Pro Gly Gln Gln Gly Pro Ser Ala
        435                 440                 445

Ser Ala Ala Ala Ala Ala Gly Gln Tyr Gly Ser Gly Pro Gly Gln Tyr
    450                 455                 460

Gly Pro Tyr Gly Pro Gly Gln Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gln Tyr Gly Pro Gly Gln Gln Gly Pro Tyr Gly Pro
            500                 505                 510

Gly Gln Ser Ala Ala Ala Ala Ala Gly Pro Gly Ser Gly Gln Tyr Gly
        515                 520                 525

Pro Gly Ala Ser Ala Ala Ala Ala Ala Gly Gln Asn Gly Pro Gly Ser
    530                 535                 540

Gly Gln Tyr Gly Pro Gly Gln Gln Gly Pro Gly Gln Ser Ala Ala Ala
545                 550                 555                 560

Ala Ala Gly Gln Tyr Gln Gln Gly Pro Gly Gln Gln Gly Pro Tyr Gly
                565                 570                 575

Pro Gly Ala Ser Ala Ala Ala Ala Ala Gly Pro Gly Ser Gly Gln Gln
            580                 585                 590

Gly Pro Gly Ala Ser
        595

<210>  4
<211>  590
<212>  PRT
<213>  Artificial Sequence

<220>
<223>  Met-PRT410

<400>  4

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly

245                 250                 255

Pro Tyr Gly Pro Gly Ala Ser Gly Gln Asn Gly Pro Gly Ser Gly Gln
        260                 265                 270

Tyr Gly Pro Gly Gln Gln Gly Pro Gly Gln Ser Ala Ala Ala Ala Ala
    275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
                500                 505                 510

Ala Ala Ala Ala Ala Gly Gln Tyr Gln Gln Gly Pro Gly Gln Gln Gly
            515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
                580                 585                 590


<210>  5
<211>  12
<212>  PRT
<213>  Artificial Sequence

<220>
<223>  HisTag

<400>  5

Met His His His His His His Ser Ser Gly Ser Ser
1               5                   10


<210>  6
<211>  608
<212>  PRT
<213>  Artificial Sequence

<220>
<223>  PRT313

<400>  6

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Ala Ala Ala
        35                  40                  45

Ala Ala Gly Gly Tyr Gly Pro Gly Gly Gln Gly Pro Gly Gln Gln Gly
    50                  55                  60

Pro Gly Ser Ser Ala Ala Ala Ala Ala Gly Pro Gly Gly Tyr Gly Pro
65                  70                  75                  80

Gly Gly Gln Gly Pro Ser Ala Ser Ala Ala Ala Ala Ala Gly Pro Gly
                85                  90                  95

Ser Gly Gln Gln Gly Pro Gly Ala Ser Ala Ala Ala Ala Ala Gly Gly
            100                 105                 110

Tyr Gly Pro Gly Gly Gln Gly Pro Gly Gln Gln Gly Pro Gly Ser Ser
        115                 120                 125

Ala Ala Ala Ala Ala Gly Gly Tyr Gly Ser Gly Pro Gly Gln Gln Gly
    130                 135                 140

Pro Tyr Gly Ser Ala Ala Ala Ala Ala Gly Pro Gly Ser Gly Gly Tyr
145                 150                 155                 160

Gly Gln Gly Pro Tyr Gly Pro Gly Ala Ser Ala Ala Ala Ala Ala Gly
                165                 170                 175

Pro Gly Gly Tyr Gly Pro Gly Gly Gln Gly Pro Ser Ala Ser Ala Ala
            180                 185                 190

Ala Ala Ala Gly Ser Gly Gln Gln Gly Pro Gly Gly Tyr Gly Pro Tyr
        195                 200                 205

Ala Ser Ala Ala Ala Ala Ala Gly Gly Tyr Gly Ser Gly Pro Gly Gln
    210                 215                 220

Gln Gly Pro Tyr Gly Pro Gly Gly Ser Ala Ala Ala Ala Ala Gly Ser
225                 230                 235                 240

Gly Gln Gln Gly Pro Gly Gln Gln Gly Pro Tyr Ala Ser Ala Ala Ala
                245                 250                 255

Ala Ala Gly Pro Gly Gly Gln Gly Pro Tyr Gly Pro Gly Ser Ser Ala
            260                 265                 270

Ala Ala Ala Ala Gly Gly Tyr Gly Tyr Gly Pro Gly Gly Gln Gly Pro
        275                 280                 285

Tyr Gly Pro Gly Ala Ser Ala Ala Ala Ala Ala Gly Gly Asn Gly Pro
    290                 295                 300

Gly Ser Gly Gly Tyr Gly Pro Gly Gln Gln Gly Pro Gly Gly Ser Ala
305                 310                 315                 320

Ala Ala Ala Ala Gly Pro Gly Gly Gln Gly Pro Tyr Gly Pro Gly Ala
                325                 330                 335

Ser Ala Ala Ala Ala Ala Gly Gly Tyr Gly Pro Gly Gly Gln Gly Pro
            340                 345                 350

Gly Gly Tyr Gly Pro Gly Ser Ser Ala Ala Ala Ala Ala Gly Pro Gly
        355                 360                 365

Gly Gln Gly Pro Tyr Gly Pro Gly Ser Ser Ala Ala Ala Ala Ala Gly
    370                 375                 380

Gly Tyr Gly Pro Gly Gln Gln Gly Pro Tyr Gly Pro Gly Gly Ser Ala
385                 390                 395                 400

Ala Ala Ala Ala Gly Gly Tyr Gln Gln Gly Pro Gly Gly Gln Gly Pro
                405                 410                 415

Tyr Gly Pro Gly Ala Ser Ala Ala Ala Ala Ala Gly Pro Gly Gly Gln
            420                 425                 430

Gly Pro Tyr Gly Pro Gly Ala Ser Ala Ala Ala Ala Ala Gly Pro Gly
        435                 440                 445

Gly Tyr Gly Pro Gly Gly Gln Gly Pro Ser Ala Ser Ala Ala Ala Ala
    450                 455                 460

Ala Gly Gly Tyr Gly Ser Gly Pro Gly Gly Tyr Gly Pro Tyr Gly Pro
465                 470                 475                 480

Gly Gly Ser Ala Ala Ala Ala Ala Gly Pro Gly Ser Gly Gln Gln Gly
                485                 490                 495

Gln Gly Pro Tyr Gly Pro Gly Ala Ser Ala Ala Ala Ala Ala Gly Gly
            500                 505                 510

Tyr Gly Pro Gly Gln Gln Gly Pro Tyr Gly Pro Gly Gly Ser Ala Ala
        515                 520                 525

Ala Ala Ala Gly Pro Gly Ser Gly Gly Tyr Gly Pro Gly Ala Ser Ala
    530                 535                 540

Ala Ala Ala Ala Gly Gly Asn Gly Pro Gly Ser Gly Gly Tyr Gly Pro
545                 550                 555                 560

Gly Gln Gln Gly Pro Gly Gly Ser Ala Ala Ala Ala Ala Gly Gly Tyr

565                 570                 575

Gln Gln Gly Pro Gly Gly Gln Gly Pro Tyr Gly Pro Gly Ala Ser Ala
            580                 585                 590

Ala Ala Ala Ala Gly Pro Gly Ser Gly Gln Gln Gly Pro Gly Ala Ser
        595                 600                 605

<210>  7
<211>  601
<212>  PRT
<213>  Artificial Sequence

<220>
<223>  PRT399

<400>  7

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Gly Gly Tyr
        35                  40                  45

Gly Pro Gly Gly Gln Gly Pro Gly Gln Gln Gly Pro Gly Ser Ser Ala
    50                  55                  60

Ala Ala Ala Ala Gly Pro Gly Gly Tyr Gly Pro Gly Gly Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gly Tyr Gly Pro Gly Gly Gln Gly Pro Gly Gln
            100                 105                 110

Gln Gly Pro Gly Ser Ser Ala Ala Ala Ala Ala Gly Gly Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gly Tyr Gly Gln Gly Pro Tyr Gly Pro Gly Ala Ser
145                 150                 155                 160

Gly Pro Gly Gly Tyr Gly Pro Gly Gly Gln Gly Pro Ser Ala Ser Ala
                165                 170                 175

Ala Ala Ala Ala Gly Ser Gly Gln Gln Gly Pro Gly Gly Tyr Gly Pro
            180                 185                 190

Tyr Ala Ser Ala Ala Ala Ala Ala Gly Gly Tyr Gly Ser Gly Pro Gly
        195                 200                 205

Gln Gln Gly Pro Tyr Gly Pro Gly Gly Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gly Gln Gly Pro Tyr Gly Pro Gly Ser Ser Ala Ala Ala Ala Ala
                245                 250                 255

Gly Gly Tyr Gly Tyr Gly Pro Gly Gly Gln Gly Pro Tyr Gly Pro Gly
            260                 265                 270

Ala Ser Gly Gly Asn Gly Pro Gly Ser Gly Gly Tyr Gly Pro Gly Gln
        275                 280                 285

Gln Gly Pro Gly Gly Ser Ala Ala Ala Ala Ala Gly Pro Gly Gly Gln
    290                 295                 300

Gly Pro Tyr Gly Pro Gly Ala Ser Ala Ala Ala Ala Ala Gly Gly Tyr
305                 310                 315                 320

Gly Pro Gly Gly Gln Gly Pro Gly Gly Tyr Gly Pro Gly Ser Ser Gly
                325                 330                 335

Pro Gly Gly Gln Gly Pro Tyr Gly Pro Gly Ser Ser Ala Ala Ala Ala
            340                 345                 350

Ala Gly Gly Tyr Gly Pro Gly Gln Gln Gly Pro Tyr Gly Pro Gly Gly
        355                 360                 365

Ser Ala Ala Ala Ala Ala Gly Gly Tyr Gln Gln Gly Pro Gly Gly Gln
    370                 375                 380

Gly Pro Tyr Gly Pro Gly Ala Ser Gly Pro Gly Gly Gln Gly Pro Tyr
385                 390                 395                 400

Gly Pro Gly Ala Ser Ala Ala Ala Ala Ala Gly Pro Gly Gly Tyr Gly
                405                 410                 415

Pro Gly Gly Gln Gly Pro Ser Ala Ser Ala Ala Ala Ala Ala Gly Gly

            420                 425                 430

Tyr Gly Ser Gly Pro Gly Gly Tyr Gly Pro Tyr Gly Pro Gly Gly Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gly Tyr Gly Pro Gly Gln Gln Gly Pro
465                 470                 475                 480

Tyr Gly Pro Gly Gly Ser Ala Ala Ala Ala Ala Gly Pro Gly Ser Gly
                485                 490                 495

Gly Tyr Gly Pro Gly Ala Ser Gly Gly Asn Gly Pro Gly Ser Gly Gly
            500                 505                 510

Tyr Gly Pro Gly Gln Gln Gly Pro Gly Gly Ser Ala Ala Ala Ala Ala
        515                 520                 525

Gly Gly Tyr Gln Gln Gly Pro Gly Gly Gln Gly Pro Tyr Gly Pro Gly
    530                 535                 540

Ala Ser Ala Ala Ala Ala Ala Gly Gly Tyr Gly Ser Gly Pro Gly Gln
545                 550                 555                 560

Gln Gly Pro Tyr Gly Pro Gly Gly Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210> 8
<211> 608
<212> PRT
<213> Artificial Sequence

<220>
<223> PRT380

<400> 8

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Ala Ala Ala
        35                  40                  45

Ala Ala Gly Gln Tyr Gly Pro Gly Gln Gln Gly Pro Gly Gln Gln Gly
    50                  55                  60

Pro Gly Ser Ser Ala Ala Ala Ala Ala Gly Pro Gly Gln Tyr Gly Pro
65                  70                  75                  80

Gly Gln Gln Gly Pro Ser Ala Ser Ala Ala Ala Ala Ala Gly Pro Gly
                85                  90                  95

Ser Gly Gln Gln Gly Pro Gly Ala Ser Ala Ala Ala Ala Ala Gly Gln
            100                 105                 110

Tyr Gly Pro Gly Gln Gln Gly Pro Gly Gln Gln Gly Pro Gly Ser Ser
        115                 120                 125

Ala Ala Ala Ala Ala Gly Gln Tyr Gly Ser Gly Pro Gly Gln Gln Gly
    130                 135                 140

Pro Tyr Gly Ser Ala Ala Ala Ala Ala Gly Pro Gly Ser Gly Gln Tyr
145                 150                 155                 160

Gly Gln Gly Pro Tyr Gly Pro Gly Ala Ser Ala Ala Ala Ala Ala Gly
                165                 170                 175

Pro Gly Gln Tyr Gly Pro Gly Gln Gln Gly Pro Ser Ala Ser Ala Ala
            180                 185                 190

Ala Ala Ala Gly Ser Gly Gln Gln Gly Pro Gly Gln Tyr Gly Pro Tyr
        195                 200                 205

Ala Ser Ala Ala Ala Ala Ala Gly Gln Tyr Gly Ser Gly Pro Gly Gln
    210                 215                 220

Gln Gly Pro Tyr Gly Pro Gly Gln Ser Ala Ala Ala Ala Ala Gly Ser
225                 230                 235                 240

Gly Gln Gln Gly Pro Gly Gln Gln Gly Pro Tyr Ala Ser Ala Ala Ala
                245                 250                 255

Ala Ala Gly Pro Gly Gln Gln Gly Pro Tyr Gly Pro Gly Ser Ser Ala
            260                 265                 270

Ala Ala Ala Ala Gly Gln Tyr Gly Tyr Gly Pro Gly Gln Gln Gly Pro

275                 280                 285

Tyr Gly Pro Gly Ala Ser Ala Ala Ala Ala Ala Gly Gln Asn Gly Pro
    290                 295                 300

Gly Ser Gly Gln Tyr Gly Pro Gly Gln Gln Gly Pro Gly Gln Ser Ala
305                 310                 315                 320

Ala Ala Ala Ala Gly Pro Gly Gln Gln Gly Pro Tyr Gly Pro Gly Ala
                325                 330                 335

Ser Ala Ala Ala Ala Ala Gly Gln Tyr Gly Pro Gly Gln Gln Gly Pro
            340                 345                 350

Gly Gln Tyr Gly Pro Gly Ser Ser Ala Ala Ala Ala Ala Gly Pro Gly
        355                 360                 365

Gln Gln Gly Pro Tyr Gly Pro Gly Ser Ser Ala Ala Ala Ala Ala Gly
    370                 375                 380

Gln Tyr Gly Pro Gly Gln Gln Gly Pro Tyr Gly Pro Gly Gln Ser Ala
385                 390                 395                 400

Ala Ala Ala Ala Gly Gln Tyr Gln Gln Gly Pro Gly Gln Gln Gly Pro
                405                 410                 415

Tyr Gly Pro Gly Ala Ser Ala Ala Ala Ala Ala Gly Pro Gly Gln Gln
            420                 425                 430

Gly Pro Tyr Gly Pro Gly Ala Ser Ala Ala Ala Ala Ala Gly Pro Gly
        435                 440                 445

Gln Tyr Gly Pro Gly Gln Gln Gly Pro Ser Ala Ser Ala Ala Ala Ala
    450                 455                 460

Ala Gly Gln Tyr Gly Ser Gly Pro Gly Gln Tyr Gly Pro Tyr Gly Pro
465                 470                 475                 480

Gly Gln Ser Ala Ala Ala Ala Ala Gly Pro Gly Ser Gly Gln Gln Gly
                485                 490                 495

Gln Gly Pro Tyr Gly Pro Gly Ala Ser Ala Ala Ala Ala Ala Gly Gln
            500                 505                 510

Tyr Gly Pro Gly Gln Gln Gly Pro Tyr Gly Pro Gly Gln Ser Ala Ala
        515                 520                 525

Ala Ala Ala Gly Pro Gly Ser Gly Gln Tyr Gly Pro Gly Ala Ser Ala
    530                 535                 540

Ala Ala Ala Ala Gly Gln Asn Gly Pro Gly Ser Gly Gln Tyr Gly Pro
545                 550                 555                 560

Gly Gln Gln Gly Pro Gly Gln Ser Ala Ala Ala Ala Ala Gly Gln Tyr
                565                 570                 575

Gln Gln Gly Pro Gly Gln Gln Gly Pro Tyr Gly Pro Gly Ala Ser Ala
            580                 585                 590

Ala Ala Ala Ala Gly Pro Gly Ser Gly Gln Gln Gly Pro Gly Ala Ser
        595                 600                 605

<210>  9
<211>  601
<212>  PRT
<213>  Artificial Sequence

<220>
<223>  PRT410

<400>  9
   Met His His His His His His Ser Ser Gly Ser Ser Gly Pro Gly Gln
   1               5                   10                  15
   Gln Gly Pro Tyr Gly Pro Gly Ala Ser Ala Ala Ala Ala Ala Gly Gln
               20                  25                  30
   Asn Gly Pro Gly Ser Gly Gln Gln Gly Pro Gly Gln Ser Gly Gln Tyr
           35                  40                  45
   Gly Pro Gly Gln Gln Gly Pro Gly Gln Gln Gly Pro Gly Ser Ser Ala
       50                  55                  60
   Ala Ala Ala Ala Gly Pro Gly Gln Tyr Gly Pro Gly Gln Gln Gly Pro
   65                  70                  75                  80
   Ser Ala Ser Ala Ala Ala Ala Ala Gly Pro Gly Ser Gly Gln Gln Gly
                   85                  90                  95
   Pro Gly Ala Ser Gly Gln Tyr Gly Pro Gly Gln Gln Gly Pro Gly Gln
               100                 105                 110
   Gln Gly Pro Gly Ser Ser Ala Ala Ala Ala Ala Gly Gln Tyr Gly Ser
           115                 120                 125
   Gly Pro Gly Gln Gln Gly Pro Tyr Gly Ser Ala Ala Ala Ala Ala Gly
   130 135 140
   Pro Gly Ser Gly Gln Tyr Gly Gln Gly Pro Tyr Gly Pro Gly Ala Ser
   145 150 155 160
   Gly Pro Gly Gln Tyr Gly Pro Gly Gln Gln Gly Pro Ser Ala Ser Ala
   165 170 175
   Ala Ala Ala Ala Gly Ser Gly Gln Gln Gly Pro Gly Gln Tyr Gly Pro
   180 185 190
   Tyr Ala Ser Ala Ala Ala Ala Ala Gly Gln Tyr Gly Ser Gly Pro Gly
   195 200 205
   Gln Gln Gly Pro Tyr Gly Pro Gly Gln Ser Gly Ser Gly Gln Gln Gly
   210 215 220
   Pro Gly Gln Gln Gly Pro Tyr Ala Ser Ala Ala Ala Ala Ala Gly Pro
   225 230 235 240
   Gly Gln Gln Gly Pro Tyr Gly Pro Gly Ser Ser Ala Ala Ala Ala Ala
   245 250 255
   Gly Gln Tyr Gly Tyr Gly Pro Gly Gln Gln Gly Pro Tyr Gly Pro Gly
   260 265 270
   Ala Ser Gly Gln Asn Gly Pro Gly Ser Gly Gln Tyr Gly Pro Gly Gln
   275 280 285
   Gln Gly Pro Gly Gln Ser Ala Ala Ala Ala Ala Gly Pro Gly Gln Gln
   290 295 300
   Gly Pro Tyr Gly Pro Gly Ala Ser Ala Ala Ala Ala Ala Gly Gln Tyr
   305 310 315 320
   Gly Pro Gly Gln Gln Gly Pro Gly Gln Tyr Gly Pro Gly Ser Ser Gly
   325 330 335
   Pro Gly Gln Gln Gly Pro Tyr Gly Pro Gly Ser Ser Ala Ala Ala Ala
   340 345 350
   Ala Gly Gln Tyr Gly Pro Gly Gln Gln Gly Pro Tyr Gly Pro Gly Gln
   355 360 365
   Ser Ala Ala Ala Ala Ala Gly Gln Tyr Gln Gln Gly Pro Gly Gln Gln
   370 375 380
   Gly Pro Tyr Gly Pro Gly Ala Ser Gly Pro Gly Gln Gln Gly Pro Tyr
   385                 390                 395                 400
   Gly Pro Gly Ala Ser Ala Ala Ala Ala Ala Gly Pro Gly Gln Tyr Gly
                   405                 410                 415
   Pro Gly Gln Gln Gly Pro Ser Ala Ser Ala Ala Ala Ala Ala Gly Gln
               420                 425                 430
   Tyr Gly Ser Gly Pro Gly Gln Tyr Gly Pro Tyr Gly Pro Gly Gln Ser
           435                 440                 445
   Gly Pro Gly Ser Gly Gln Gln Gly Gln Gly Pro Tyr Gly Pro Gly Ala
       450                 455                 460
   Ser Ala Ala Ala Ala Ala Gly Gln Tyr Gly Pro Gly Gln Gln Gly Pro
   465                 470                 475                 480
   Tyr Gly Pro Gly Gln Ser Ala Ala Ala Ala Ala Gly Pro Gly Ser Gly
                   485                 490                 495
   Gln Tyr Gly Pro Gly Ala Ser Gly Gln Asn Gly Pro Gly Ser Gly Gln
               500                 505                 510
   Tyr Gly Pro Gly Gln Gln Gly Pro Gly Gln Ser Ala Ala Ala Ala Ala
           515                 520                 525
   Gly Gln Tyr Gln Gln Gly Pro Gly Gln Gln Gly Pro Tyr Gly Pro Gly
       530                 535                 540
   Ala Ser Ala Ala Ala Ala Ala Gly Gln Tyr Gly Ser Gly Pro Gly Gln
   545                 550                 555                 560
   Gln Gly Pro Tyr Gly Pro Gly Gln Ser Gly Ser Gly Gln Gln Gly Pro
                   565                 570                 575
   Gly Gln Gln Gly Pro Tyr Ala Ser Ala Ala Ala Ala Ala Gly Pro Gly
               580                 585                 590
   Ser Gly Gln Gln Gly Pro Gly Ala Ser
           595                 600

<210>  10
<211>  565
<212>  PRT
<213>  Artificial Sequence

<220>
<223>  Met-PRT468

<400>  10

Met Gly Pro Gly Gln Gln Gly Pro Tyr Gly Pro Gly Ala Ser Ala Ala
1               5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
        35                  40                  45

Gln Gly Pro Gly Ser Ser Ala Ala Ala Ala Ala Ala Ala Gly Pro Gly
    50                  55                  60

Gln Tyr Gly Pro Gly Gln Gln Gly Pro Ser Ala Ser Ala Ala Ala Ala
65                  70                  75                  80

Ala Ala Ala Gly Pro Gly Ser Gly Gln Gln Gly Pro Gly Ala Ser Gly
                85                  90                  95

Gln Tyr Gly Pro Gly Gln Gln Gly Pro Gly Gln Gln Gly Pro Gly Ser
            100                 105                 110

Ser Ala Ala Ala Ala Ala Ala Ala Gly Ser Tyr Gly Ser Gly Pro Gly
        115                 120                 125

Gln Gln Gly Pro Tyr Gly Ser Ala Ala Ala Ala Ala Ala Ala Gly Pro
    130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
                165                 170                 175

Ala Ala Ala Ala Ala Gly Ser Gly Gln Gln Gly Pro Gly Gln Tyr Gly
            180                 185                 190

Pro Tyr Ala Ser Ala Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
    210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
            260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
    290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
            340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
    370                 375                 380

Ala Ala Ala Ala Ala Gly Ser Tyr Gln Gln Gly Pro Gly Gln Gln Gly
385                 390                 395                 400

Pro Tyr Gly Pro Gly Ala Ser Gly Pro Gly Gln Gln Gly Pro Tyr Gly
                405                 410                 415

Pro Gly Ala Ser Ala Ala Ala Ala Ala Ala Ala Gly Pro Gly Gln Tyr
            420                 425                 430

Gly Pro Gly Gln Gln Gly Pro Ser Ala Ser Ala Ala Ala Ala Ala Ala
        435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
                485                 490                 495

Gly Gln Gln Gly Pro Tyr Gly Pro Gly Pro Ser Ala Ala Ala Ala Ala
                500                 505                 510

Ala Ala Gly Pro Gly Ser Gly Gln Tyr Gly Pro Gly Ala Ser Gly Gln
            515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565

<210>  11
<211>  576
<212>  PRT
<213>  Artificial Sequence

<220>
<223>  PRT468

<400>  11

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Gly Pro Gly
65                  70                  75                  80

Gln Gln Gly Pro Ser Ala Ser Ala Ala Ala Ala Ala Ala Ala Gly Pro
                85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
        115                 120                 125

Ala Ala Ala Gly Ser Tyr Gly Ser Gly Pro Gly Gln Gln Gly Pro Tyr
    130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
                165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
        195                 200                 205

Ala Ala Ala Ala Ala Ala Gly Ser Tyr Gly Ser Gly Pro Gly Gln Gln
    210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Ala Gly Pro
                245                 250                 255

Gly Gln Gln Gly Pro Tyr Gly Pro Gly Ser Ser Ala Ala Ala Ala Ala
            260                 265                 270

Ala Ala Gly Ser Tyr Gly Tyr Gly Pro Gly Gln Gln Gly Pro Tyr Gly
        275                 280                 285

Pro Gly Ala Ser Gly Gln Asn Gly Pro Gly Ser Gly Gln Tyr Gly Pro
    290                 295                 300

Gly Gln Gln Gly Pro Gly Pro Ser Ala Ala Ala Ala Ala Ala Ala Gly
305                 310                 315                 320

Pro Gly Gln Gln Gly Pro Tyr Gly Pro Gly Ala Ser Ala Ala Ala Ala
                325                 330                 335

Ala Ala Ala Gly Ser Tyr Gly Pro Gly Gln Gln Gly Pro Gly Gln Tyr
            340                 345                 350

Gly Pro Gly Ser Ser Gly Pro Gly Gln Gln Gly Pro Tyr Gly Pro Gly
        355                 360                 365

Ser Ser Ala Ala Ala Ala Ala Ala Ala Gly Ser Tyr Gly Pro Gly Gln
    370                 375                 380

Gln Gly Pro Tyr Gly Pro Gly Pro Ser Ala Ala Ala Ala Ala Ala Ala
385                 390                 395                 400

Gly Ser Tyr Gln Gln Gly Pro Gly Gln Gln Gly Pro Tyr Gly Pro Gly
                405                 410                 415

Ala Ser Gly Pro Gly Gln Gln Gly Pro Tyr Gly Pro Gly Ala Ser Ala
            420                 425                 430

Ala Ala Ala Ala Ala Ala Gly Pro Gly Gln Tyr Gly Pro Gly Gln Gln
        435                 440                 445

Gly Pro Ser Ala Ser Ala Ala Ala Ala Ala Ala Ala Gly Ser Tyr Gly
    450                 455                 460

Ser Gly Pro Gly Gln Tyr Gly Pro Tyr Gly Pro Gly Gln Ser Gly Pro
465                 470                 475                 480

Gly Ser Gly Gln Gln Gly Gln Gly Pro Tyr Gly Pro Gly Ala Ser Ala
                485                 490                 495

Ala Ala Ala Ala Ala Ala Gly Ser Tyr Gly Pro Gly Gln Gln Gly Pro
            500                 505                 510

Tyr Gly Pro Gly Pro Ser Ala Ala Ala Ala Ala Ala Ala Gly Pro Gly
        515                 520                 525

Ser Gly Gln Tyr Gly Pro Gly Ala Ser Gly Gln Asn Gly Pro Gly Ser
    530                 535                 540

Gly Gln Tyr Gly Pro Gly Gln Gln Gly Pro Gly Pro Ser Ala Ala Ala
545                 550                 555                 560

Ala Ala Ala Ala Gly Pro Gly Ser Gly Gln Gln Gly Pro Gly Ala Ser
                565                 570                 575

<210>  12
<211>  601
<212>  PRT
<213>  Artificial Sequence

<220>
<223>  PRT410

<400>  12

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  13
<211>  252
<212>  PRT
<213>  Artificial Sequence

<220>
<223>  Collagen-type4-Kai

<400>  13

Met His His His His His His Ser Ser Gly Ser Ser Lys Asp Gly Val
1               5                   10                  15

Pro Gly Phe Pro Gly Ser Glu Gly Val Lys Gly Asn Arg Gly Phe Pro
            20                  25                  30

Gly Leu Met Gly Glu Asp Gly Ile Lys Gly Gln Lys Gly Asp Ile Gly
        35                  40                  45

Pro Pro Gly Phe Arg Gly Pro Thr Glu Tyr Tyr Asp Thr Tyr Gln Glu
    50                  55                  60

Lys Gly Asp Glu Gly Thr Pro Gly Pro Pro Gly Pro Arg Gly Ala Arg
65                  70                  75                  80

Gly Pro Gln Gly Pro Ser Gly Pro Pro Gly Val Pro Gly Ser Pro Gly
                85                  90                  95

Ser Ser Arg Pro Gly Leu Arg Gly Ala Pro Gly Trp Pro Gly Leu Lys
            100                 105                 110

Gly Ser Lys Gly Glu Arg Gly Arg Pro Gly Lys Asp Ala Met Gly Thr
        115                 120                 125

Pro Gly Ser Pro Gly Cys Ala Gly Ser Pro Gly Leu Pro Gly Ser Pro
    130                 135                 140

Gly Pro Pro Gly Pro Pro Gly Asp Ile Val Phe Arg Lys Gly Pro Pro
145                 150                 155                 160

Gly Asp His Gly Leu Pro Gly Tyr Leu Gly Ser Pro Gly Ile Pro Gly
                165                 170                 175

Val Asp Gly Pro Lys Gly Glu Pro Gly Leu Leu Cys Thr Gln Cys Pro
            180                 185                 190

Tyr Ile Pro Gly Pro Pro Gly Leu Pro Gly Leu Pro Gly Leu His Gly
        195                 200                 205

Val Lys Gly Ile Pro Gly Arg Gln Gly Ala Ala Gly Leu Lys Gly Ser
    210                 215                 220

Pro Gly Ser Pro Gly Asn Thr Gly Leu Pro Gly Phe Pro Gly Phe Pro
225                 230                 235                 240

Gly Ala Gln Gly Asp Pro Gly Leu Lys Gly Glu Lys
                245                 250

<210>  14
<211>  310
<212>  PRT
<213>  Artificial Sequence

<220>
<223>  Resilin-Kai

<400>  14

Met His His His His His His Pro Glu Pro Pro Val Asn Ser Tyr Leu
1               5                   10                  15

Pro Pro Ser Asp Ser Tyr Gly Ala Pro Gly Gln Ser Gly Pro Gly Gly
            20                  25                  30

Arg Pro Ser Asp Ser Tyr Gly Ala Pro Gly Gly Gly Asn Gly Gly Arg
        35                  40                  45

Pro Ser Asp Ser Tyr Gly Ala Pro Gly Gln Gly Gln Gly Gln Gly Gln
    50                  55                  60

Gly Gln Gly Gly Tyr Ala Gly Lys Pro Ser Asp Ser Tyr Gly Ala Pro
65                  70                  75                  80

Gly Gly Gly Asp Gly Asn Gly Gly Arg Pro Ser Ser Ser Tyr Gly Ala
                85                  90                  95

Pro Gly Gly Gly Asn Gly Gly Arg Pro Ser Asp Thr Tyr Gly Ala Pro
            100                 105                 110

Gly Gly Gly Asn Gly Gly Arg Pro Ser Asp Thr Tyr Gly Ala Pro Gly
        115                 120                 125

Gly Gly Gly Asn Gly Asn Gly Gly Arg Pro Ser Ser Ser Tyr Gly Ala
    130                 135                 140

Pro Gly Gln Gly Gln Gly Asn Gly Asn Gly Gly Arg Pro Ser Ser Ser
145                 150                 155                 160

Tyr Gly Ala Pro Gly Gly Gly Asn Gly Gly Arg Pro Ser Asp Thr Tyr
                165                 170                 175

Gly Ala Pro Gly Gly Gly Asn Gly Gly Arg Pro Ser Asp Thr Tyr Gly
            180                 185                 190

Ala Pro Gly Gly Gly Asn Asn Gly Gly Arg Pro Ser Ser Ser Tyr Gly
        195                 200                 205

Ala Pro Gly Gly Gly Asn Gly Gly Arg Pro Ser Asp Thr Tyr Gly Ala
    210                 215                 220

Pro Gly Gly Gly Asn Gly Asn Gly Ser Gly Gly Arg Pro Ser Ser Ser
225                 230                 235                 240

Tyr Gly Ala Pro Gly Gln Gly Gln Gly Gly Phe Gly Gly Arg Pro Ser
                245                 250                 255

Asp Ser Tyr Gly Ala Pro Gly Gln Asn Gln Lys Pro Ser Asp Ser Tyr
            260                 265                 270

Gly Ala Pro Gly Ser Gly Asn Gly Asn Gly Gly Arg Pro Ser Ser Ser
        275                 280                 285

Tyr Gly Ala Pro Gly Ser Gly Pro Gly Gly Arg Pro Ser Asp Ser Tyr
    290                 295                 300

Gly Pro Pro Ala Ser Gly
305                 310

<210>  15
<211>  282
<212>  PRT

<213>  Artificial Sequence

<220>
<223>  elastin short

<400>  15

Met His His His His His His Ser Ser Gly Ser Ser Leu Gly Val Ser
1               5                   10                  15

Ala Gly Ala Val Val Pro Gln Pro Gly Ala Gly Val Lys Pro Gly Lys
            20                  25                  30

Val Pro Gly Val Gly Leu Pro Gly Val Tyr Pro Gly Gly Val Leu Pro
        35                  40                  45

Gly Ala Arg Phe Pro Gly Val Gly Val Leu Pro Gly Val Pro Thr Gly
    50                  55                  60

Ala Gly Val Lys Pro Lys Ala Pro Gly Val Gly Gly Ala Phe Ala Gly
65                  70                  75                  80

Ile Pro Gly Val Gly Pro Phe Gly Gly Pro Gln Pro Gly Val Pro Leu
                85                  90                  95

Gly Tyr Pro Ile Lys Ala Pro Lys Leu Pro Gly Gly Tyr Gly Leu Pro
            100                 105                 110

Tyr Thr Thr Gly Lys Leu Pro Tyr Gly Tyr Gly Pro Gly Gly Val Ala
        115                 120                 125

Gly Ala Ala Gly Lys Ala Gly Tyr Pro Thr Gly Thr Gly Val Gly Pro
    130                 135                 140

Gln Ala Ala Ala Ala Ala Ala Ala Lys Ala Ala Ala Lys Phe Gly Ala
145                 150                 155                 160

Gly Ala Ala Gly Val Leu Pro Gly Val Gly Gly Ala Gly Val Pro Gly
                165                 170                 175

Val Pro Gly Ala Ile Pro Gly Ile Gly Gly Ile Ala Gly Val Gly Thr
            180                 185                 190

Pro Ala Ala Ala Ala Ala Ala Ala Ala Ala Ala Lys Ala Ala Lys Tyr
        195                 200                 205

Gly Ala Ala Ala Gly Leu Val Pro Gly Gly Pro Gly Phe Gly Pro Gly
    210                 215                 220

Val Val Gly Val Pro Gly Ala Gly Val Pro Gly Val Gly Val Pro Gly
225                 230                 235                 240

Ala Gly Ile Pro Val Val Pro Gly Ala Gly Ile Pro Gly Ala Ala Val
                245                 250                 255

Pro Gly Val Val Ser Pro Glu Ala Ala Ala Lys Ala Ala Ala Lys Ala
            260                 265                 270

Ala Lys Tyr Gly Ala Arg Pro Gly Val Gly
        275                 280

<210>  16
<211>  468
<212>  PRT
<213>  Artificial Sequence

<220>
<223>  type I keratin 26

<400>  16

Met Ser Phe Arg Leu Ser Gly Val Ser Arg Arg Leu Cys Ser Gln Ala
1               5                   10                  15

Gly Thr Gly Arg Leu Thr Gly Gly Arg Thr Gly Phe Arg Ala Gly Asn
            20                  25                  30

Val Cys Ser Gly Leu Gly Ala Gly Ser Ser Phe Ser Gly Pro Leu Gly
        35                  40                  45

Ser Val Ser Ser Lys Gly Ser Phe Ser His Gly Gly Gly Gly Leu Gly
    50                  55                  60

Ser Gly Val Cys Thr Gly Phe Leu Glu Asn Glu His Gly Leu Leu Pro
65                  70                  75                  80

Gly Asn Glu Lys Val Thr Leu Gln Asn Leu Asn Asp Arg Leu Ala Ser
                85                  90                  95

Tyr Leu Asp His Val Cys Thr Leu Glu Glu Ala Asn Ala Asp Leu Glu
            100                 105                 110

Gln Lys Ile Lys Gly Trp Tyr Glu Lys Tyr Gly Pro Gly Ser Gly Arg
        115                 120                 125

Gln Leu Ala His Asp Tyr Ser Lys Tyr Phe Ser Val Thr Glu Asp Leu
    130                 135                 140

Lys Arg Gln Ile Ile Ser Val Thr Thr Cys Asn Ala Ser Ile Val Leu
145                 150                 155                 160

Gln Asn Glu Asn Ala Arg Leu Thr Ala Asp Asp Phe Arg Leu Lys Cys
                165                 170                 175

Glu Asn Glu Leu Ala Leu His Gln Ser Val Glu Ala Asp Ile Asn Gly
            180                 185                 190

Leu His Arg Val Met Asp Glu Leu Thr Leu Cys Thr Ser Asp Leu Glu
        195                 200                 205

Met Gln Cys Glu Ala Leu Ser Glu Glu Leu Thr Tyr Leu Lys Lys Asn
    210                 215                 220

His Gln Glu Glu Met Lys Val Met Gln Gly Ala Ala Arg Gly Asn Val
225                 230                 235                 240

Asn Val Glu Ile Asn Ala Ala Pro Gly Val Asp Leu Thr Val Leu Leu
                245                 250                 255

Asn Asn Met Arg Ala Glu Tyr Glu Asp Leu Ala Glu Gln Asn His Glu
            260                 265                 270

Asp Ala Glu Ala Trp Phe Ser Glu Lys Ser Thr Ser Leu His Gln Gln
        275                 280                 285

Ile Ser Asp Asp Ala Gly Ala Ala Met Ala Ala Arg Asn Glu Leu Met
    290                 295                 300

Glu Leu Lys Arg Asn Leu Gln Thr Leu Glu Ile Glu Leu Gln Ser Leu
305                 310                 315                 320

Leu Ala Met Lys His Ser Tyr Glu Cys Ser Leu Ala Glu Thr Glu Ser
                325                 330                 335

Asn Tyr Cys His Gln Leu Gln Gln Ile Gln Glu Gln Ile Gly Ala Met
            340                 345                 350

Glu Asp Gln Leu Gln Gln Ile Arg Met Glu Thr Glu Gly Gln Lys Leu
        355                 360                 365

Glu His Glu Arg Leu Leu Asp Val Lys Ile Phe Leu Glu Lys Glu Ile
    370                 375                 380

Glu Met Tyr Cys Lys Leu Ile Asp Gly Glu Gly Arg Lys Ser Lys Ser
385                 390                 395                 400

Thr Cys Tyr Lys Ser Glu Gly Arg Gly Pro Lys Asn Ser Glu Asn Gln

405                 410                 415

Val Lys Asp Ser Lys Glu Glu Ala Val Val Lys Thr Val Val Gly Glu
            420                 425                 430

Leu Asp Gln Leu Gly Ser Val Leu Ser Leu Arg Val His Ser Val Glu
        435                 440                 445

Glu Lys Ser Ser Lys Ile Ser Asn Ile Thr Met Glu Gln Arg Leu Pro
    450                 455                 460

Ser Lys Val Pro
465

## Description

### Technical Field

The present invention relates to a molded article and a method for producing a molded article.

### Background Art

Attempts are being made to replace metallic materials with organic materials for the purpose of weight reduction, cost reduction, the facilitation of molding, and the like. As such organic materials, phenolic resins having a high hardness are often used, and, in order to further increase the bending elastic modulus or the bending strength, a method in which a phenolic resin fiber is added to a matrix resin containing a phenolic resin is known (for example, refer to Patent Literature 1). In addition, due to a rising demand for environmental protection, bioplastics which are non-petroleum-based materials have been drawing attention, and, for example, it is known that a molded article having a bending elastic modulus of 4.5 GPa can be obtained by resinifying silk powder (for example, refer to Non-Patent Literature 1).

### Citation List

### Patent Literature

[Patent Literature 1] JP 2014-80491 A

### Non Patent Literature

[Non-Patent Literature 1] Shinji Hirai, Function & Materials, June 2014, "Environment-conscious silk and wool resins using waste-derived animal protein"
[Non-Patent Literature 2] Science, 2002, Vol. 295, pp. 472 to 476

### Summary of Invention

### Technical Problem

However, the material as disclosed by Patent Literature 1 is a material exhibiting a stiffening effect by containing a fiber (reinforcement fiber) in the matrix resin, and it is difficult to achieve a high strength with the matrix resin alone. In addition, it is not possible to obtain a molded article having a bending elastic modulus of more than 4.5 GPa using a biodegradable material.

Therefore, an object of the present invention is to provide a biodegradable molded article having an excellent bending elastic modulus and an excellent bending strength and a method for producing the same.

### Solution to Problem

The present invention provides the following [1] to [9].
[1] A method for producing a molded article, comprising a step 1 of adding water to a composition including a structural protein, a step 2 of molding a water-containing composition obtained in the step 1, and a step 3 of drying the molded composition obtained in the step 2 to obtain the molded article.
[2] The method according to [1], in which the step 2 comprises hot pressing.
[3] The method according to [1] or [2], in which the structural protein comprises a spider silk protein or a recombinant polypeptide thereof.
[4] A molded article which is obtained by using the method according to any one of [1] to [3].
[5] A molded article which is a molded article of a composition including a structural protein and has a bending elastic modulus of more than 6.9 GPa.
[6] The molded article according to [5] which is a hot press-molded article.
[7] The molded article according to [5] or [6], in which the structural protein includes a spider silk protein or a recombinant polypeptide thereof.
[8] The molded article according to any one of [5] to [7], in which a bending strength is 140 MPa or more.
[9] The molded article according to any one of [5] to [8], in which a Vickers hardness is 45 HV or more.

The molded article has characteristics of being biodegradable, being obtained from a structural protein and/or a recombinant polypeptide thereof as a raw material, and being formed by molding the raw material. The molded article exhibits an extremely high bending elastic modulus (for example, more than 6.9 GPa) even without using an additive material such as a reinforced fiber due to the above-described characteristics. Furthermore, the molded article can also be imparted with transparency and thus has an advantage of having a range of applicable uses which can be extremely broadening compared with resins which have a high strength but are not transparent such as a phenolic resin or polyether ether ketone (PEEK). In addition, the structural protein allows a variety of inheritable genetic modifications, and thus it is possible to easily optimize the performance in accordance with the final uses.

In a manufacturing process of the molded article, water is added to the composition, and the composition is molded and then dried, whereby a molded article having an excellent bending elastic modulus and an excellent bending strength is produced. The molded article refers to an article or the like molded using a casting mold (mold), but hot pressing provides a superior bending elastic modulus to the molded article.

### Advantageous Effects of Invention

According to the present invention, a biodegradable molded article exhibiting an excellent bending elastic modulus and an excellent bending strength even without using an additive material such as a reinforced fiber and a method for producing the same are provided.

### Brief Description of Drawings

Fig. 1 is a schematic cross-sectional view of a press molding machine.
Fig. 2 illustrates schematic cross-sectional views of the press molding machine (a) before the introduction of a composition, (b) immediately after the introduction of the composition, and (c) with the composition in a state of being hot pressed.

### Description of Embodiments

Hereinafter, a preferred embodiment of the present invention will be described. However, the present invention is not limited to the following embodiment by any means.

A molded article according to the present embodiment is obtained by introducing a composition including a structural protein to a casting mold (mold) and molding the composition.

### [Structural protein]

The structural protein refers to a protein having a role of building a living structure and is different from a functional protein such as an enzyme, a hormone, or an antibody. Examples of the structural protein include natural structural proteins such as fibroin, collagen, resilin, elastin, and keratin which occur in nature. As fibroin occurring in nature, fibroin produced by insects and spiders are known. In the present embodiment, the structural protein preferably includes a spider silk protein.

Examples of the fibroin produced by insects include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and hornet silk proteins discharged by the larvae of Vespa simillima xanthoptera.

More specific examples of the fibroin produced by insects include silkworm fibroin L chain (GenBank accession number M76430 (base sequence), AAA27840.1 (amino-acid sequence)).

Spiders have a maximum of seven types of silk glands from which various types of fibroin (spider silk protein) having different properties are produced respectively. Spider silk protein is called with various names of major ampullate spider protein (MaSp) having a high toughness, minor ampullate spider protein (MiSp) having a high elongation power, flagelliform (Flag), tubuliform, aggregate, aciniform, and pyriform depending on the source organs.

Examples of the fibroin produced by spiders include spider silk proteins produced by spiders belonging to Araneus such as Araneus ventricosus, diadem spiders, Araneus pinguis, Araneus pentagrammicus, and Araneus nojimai, spiders belonging to Neoscona such as Neoscona Scylla, Neoscona nautica, Neoscona adianta, and Neoscona scylloides, spiders belonging to Pronus such as Pronous minutus, spiders belonging to Cyrtarachne such as Cyrtarachne bufo and Gyrtarachne inaequalis, spiders belonging to Gasteracantha such as Gasteracantha kuhlii and Gasteracantha mammosa, spiders belonging to Ordgarius such as Ordgarius hobsoni and Ordgarius sexspinosus, spiders belonging to Argiope such as Argiope amoena, Argiope minuta, and Argiope bruennichii, spiders belonging to Arachnura such as Arachnura logio Yaginuma, spiders belonging to Acusilas such as Acusilas coccineus, spiders belonging to Cytophora such as Cyrtophora moluccensis, Cyrtophora exanthematica, and Cyrtophora unicolor, spiders belonging to Poltys such as Poltys illepidus, spiders belonging to Cyclosa such as Cyclosa octotuberculata, Cyclosa sedeculata Karsch, Cyclosa vallata, and Cyclosa atrata, and spiders belonging to Chorizopes such as Chorizopes nipponicus and spider silk proteins produced by spiders belonging to Tetragnatha such as Tetragnatha praedonia, Tetragnatha maxillosa, Araneus viridiventris Yaginuma, and Tetragnatha squamata, spiders belonging to Leucauge such as Leucauge magnifica, Leucauge blanda, and Leucauge subblanda, spiders belonging to Nephila such as Nephila clavata and Nephila pilipes, spiders belonging to Menosira such as Menosira ornate, spiders belonging to Dyschiriognatha such as Dyschiriognatha tenera, spiders belonging to Latrodectus such as Latrodectus mactans, Latrodectus hasselti, Latrodectus geometricus, and Latrodectus tredecimguttatus, and spiders belonging to Tetragnathidae such as spiders belonging to Euprosthenops. Examples of the spider silk proteins include drag line proteins such as MaSp (MaSp1 and MaSp2) and ADF (ADF3 and ADF4), MiSp (MiSp1 and MiSp2), and the like.

More specific examples of the fibroin produced by spiders include fibroin-3 (adf-3) [derived from Araneus diadematus] (GenBank accession number AAC47010 (amino-acid sequence), U47855 (base sequence)), fibroin-4 (adf-4) [derived from Araneus diadematus] (GenBank accession number AAC47011 (amino-acid sequence), U47856 (base sequence)), dragline silk protein spidroin 1 [derived from Nephila clavipes] (GenBank accession number AAC04504 (amino-acid sequence), U37520 (base sequence)), major angullate spidroin 1 [derived from Latrodectus hesperus] (GenBank accession number ABR68856 (amino-acid sequence), EF595246 (base sequence)), dragline silk protein spidroin 2 [derived from Nephila clavata] (GenBank accession number AAL32472 (amino-acid sequence), AF441245 (base sequence)), major anpullate spidroin 1 [derived from Euprosthenops australis] (GenBank accession number CAJ00428 (amino-acid sequence), AJ973155 (base sequence)), and major ampullate spidroin 2 [derived from Euprosthenops australis] (GenBank accession number CAM32249.1 (amino-acid sequence), AM490169 (base sequence)), minor ampullate silk protein 1 [Nephila clavipes] (GenBank accession number AAC14589.1 (amino-acid sequence)), minor ampullate silk protein 2 [Nephila clavipes] (GenBank accession number AAC14591.1 (amino-acid sequence)), minor ampullate spidroin-like protein [Nephilengys cruentata] (GenBank accession number ABR37278.1 (amino-acid sequence)), and the like.

More specific examples of the naturally occurring fibroin further include fibroin having a SEQ ID No. registered in NCBI GenBank. The fibroin having a SEQ ID No. registered in NCBI GenBank can be confirmed by, for example, extracting, from sequences including INV as DIVISION among sequence information registered in NCBI GenBank, sequences for which spidroin, ampullate, fibroin, 'silk and polypeptide', or 'silk and protein' is described as the key word in DEFINITION, character strings of a specific product from CDS, and sequences for which a specific character string is described in TISSUE TYPE from SOURCE.

The structural protein may be a polypeptide derived from the natural structural protein, that is, a recombinant polypeptide. For example, recombinant fibroin is produced in several foreign protein production systems, and, as a method for producing the recombinant fibroin, transgenic goats, transgenic silkworms, or recombinant plant or mammalian cells are used (refer to Non-Patent Literature 2).

The recombinant fibroin can be obtained by, for example, deleting one or a plurality of sequences that code an (A)ₙ motif from the genetic sequence of a cloned naturally occurring fibroin. In addition, the recombinant fibroin can also be obtained by, for example, designing an amino-acid sequence corresponding to an amino-acid sequence of naturally occurring fibroin from which one or a plurality of (A)ₙ motifs is deleted and chemically synthesizing a nucleic acid that codes the designed amino-acid sequence. In any cases, in addition to the modification corresponding to the deletion of the (A)ₙ motifs from the amino-acid sequence of naturally occurring fibroin, furthermore, the modification of the amino-acid sequence corresponding to the substitution, deletion, insertion, and/or addition of one or a plurality of amino-acid residues may be carried out. The substitution, deletion, insertion, and/or addition of amino-acid residues can be carried out using a method well known to a person skilled in the art such as site-directed mutagenesis. Specifically, these can be carried out according to the method described in Nucleic Acid Res. 10, 6487 (1982), Methods in Enzymology, 100, 448 (1983), or the like.

A recombinant polypeptide of a major dragline silk protein can be represented as, for example, a protein including a domain sequence represented by Formula 1: [(A)ₙ motif-REP]ₘ (here, in Formula 1, the (A)ₙ motif represents an amino-acid sequence constituted of 4 to 20 amino-acid residues, and the percentage of the number of alanine residues with respect to the number of all of amino-acid residues in the (A)ₙ motif is 80% or more. REP represents an amino-acid sequence constituted of 10 to 200 amino-acid residues. m represents an integer of 8 to 300. A plurality of (A)ₙ motifs may be identical amino-acid sequences or different amino-acid sequences. A plurality of REP's may be identical amino-acid sequences or different amino-acid sequences.). Specific examples thereof include a protein including an amino-acid sequence represented by a SEQ ID No. 12.

Examples of a recombinant polypeptide of collagen include a protein including a domain sequence represented by Formula 2: [REP2]ₒ (here, in Formula 2, o represents an integer of 5 to 300. REP2 represents an amino-acid sequence constituted of Gly-X-Y, and X and Y represent arbitrary amino-acid residues other than Gly. A plurality of REP2 may be identical amino-acid sequences or different amino-acid sequences.). Specific examples thereof include a protein including an amino-acid sequence represented by a SEQ ID No. 13. The amino-acid sequence represented by the SEQ ID No. 13 is an amino-acid sequence obtained by adding an amino-acid sequence represented by a SEQ ID No. 5 (tag sequence and hinge sequence) to an N terminal of an amino-acid sequence from the 301^{st} residue through the 540^{th} residue which corresponds to the repeat portion and the motif of a partial sequence (NCBI Genbank accession number: CAA56335.1, GI: 3702452) of a collagen type 4 of a human being procured from NCBI database.

Examples of a recombinant polypeptide of resilin include a protein including a domain sequence represented by Formula 3: [REP3]ₚ (here, in Formula 3, p represents an integer of 4 to 300. REP3 represents an amino-acid sequence constituted of Ser-J-J-Tyr-Gly-U-Pro. J represents an arbitrary amino-acid residue and is particularly preferably an amino-acid residue selected from the group consisting of Asp, Ser, and Thr. U represents an arbitrary amino-acid residue and is particularly preferably an amino-acid residue selected from the group consisting of Pro, Ala, Thr, and Ser. A plurality of REP3 may be identical amino-acid sequences or different amino-acid sequences.). Specific examples thereof include a protein including an amino-acid sequence represented by a SEQ ID No. 14. The amino-acid sequence represented by the SEQ ID No. 14 is an amino-acid sequence obtained by substituting Thr of the 87^{th} residue into Ser in the amino-acid sequence of resilin (NCBI Genbank accession number: NP 611157, GI: 24654243) and adding the amino-acid sequence represented by the SEQ ID No. 5 (tag sequence and hinge sequence) to the N terminal of an amino-acid sequence from the 19^{th} residue through the 321^{st} residue of a sequence in which Asn of the 95^{th} residue is substituted into Asp.

Examples of a recombinant polypeptide of elastin include a protein having an amino-acid sequence of NCBI Genbank accession number: AAC98395 (human), 147076 (sheep), NP786966 (cow), or the like. Specific examples thereof include a protein including an amino-acid sequence represented by a SEQ ID No. 15. The amino-acid sequence represented by the SEQ ID No. 15 is an amino-acid sequence obtained by adding the amino-acid sequence represented by the SEQ ID No. 5 (tag sequence and hinge sequence) to the N terminal of an amino-acid sequence from the 121^{th} residue through the 390^{st} residue of the amino-acid sequence of NCBI Genbank accession number: AAC98395.

Examples of a recombinant polypeptide of keratin include type I keratin of Capra hircus and the like. Specific examples thereof include a protein including an amino-acid sequence represented by a SEQ ID No. 16 (an amino-acid sequence of NCBI Genbank accession number of ACY30466).

The recombinant polypeptide may be recombinant fibroin including (i) an amino-acid sequence represented by a SEQ ID No. 2, a SEQ ID No. 4, or a SEQ ID No. 10 or (ii) an amino-acid sequence having a degree of identity of 90% or more with an amino-acid sequence represented by the SEQ ID No. 2, the SEQ ID No. 4, or the SEQ ID No. 10.

The recombinant fibroin including (i) the amino-acid sequence represented by the SEQ ID No. 2, the SEQ ID No. 4, or the SEQ ID No. 10 will be described. The amino-acid sequence represented by the SEQ ID No. 2 is an amino-acid sequence obtained by deleting (A)ₙ motifs every two motifs from an amino-acid sequence (corresponding to naturally occurring fibroin) represented by a SEQ ID No. 1 which corresponds to naturally occurring fibroin from the N terminal side toward the C terminal side and further inserting one [(A)ₙ motif-REP] to the front of a C terminal sequence. The amino-acid sequence represented by the SEQ ID No. 4 is an amino-acid sequence obtained by substituting all of GGX's in REP of the amino-acid sequence represented by the SEQ ID No. 2 into GQX's. The amino-acid sequence represented by the SEQ ID No. 10 is an amino-acid sequence obtained by inserting two alanine residues into the C terminal side of individual (A)ₙ motifs in the amino-acid sequence represented by the SEQ ID No. 4, furthermore, substituting some of glutamine (Q) residues into serin (S) residues, and deleting some of amino acids on the N terminal side so as to have a molecular weight that is almost the same as the molecular weight of the SEQ ID No. 4. Meanwhile, an amino-acid sequence represented by a SEQ ID No. 3 is an amino-acid sequence obtained by substituting all of GGX's in REP of the amino-acid sequence represented by the SEQ ID No. 1 into GQX's.

The recombinant fibroin including (ii) an amino-acid sequence having 90% or more of the sequence identity with the amino-acid sequence represented by the SEQ ID No. 2, the SEQ ID No. 4, or the SEQ ID No. 10 will be described. The recombinant fibroin (ii) includes an amino-acid sequence having 90% or more of the sequence identity with the amino-acid sequence represented by the SEQ ID No. 2, the SEQ ID No. 4, or the SEQ ID No. 10. The recombinant fibroin (ii) is also a protein including a domain sequence represented by Formula 1: [(A)ₙ motif-REP]ₘ. The sequence identity is preferably 95% or more.

The above-described recombinant fibroin may include a tag sequence in any one or both of the N terminal and the C terminal. In such a case, the isolation, immobilization, detection, visualization, and the like of the recombinant fibroin become possible.

Examples of the tag sequence include an affinity tag which uses the specific affinity (binding property, affinity) to other molecules. Specific examples of the affinity tag include a histidine tag (His tag). The His tag is a short peptide in which approximately 4 to 10 histidine residues are arrayed and has a property of being specifically bound with a metal ion of nickel or the like and thus can be used for the isolation of recombinant fibroin by chelating metal chromatography. Specific examples of the tag sequence include the amino-acid sequence represented by the SEQ ID No. 5 (amino-acid sequence including the His tag).

In addition, it is also possible to use tag sequences such as glutathione-S-transferase (GST) which is specifically bound with glutathione and maltose-binding protein (MBP) which is specifically bound with maltose.

Furthermore, it is also possible to use an "epitope tag" which uses an antigen-antibody reaction. When a peptide exhibiting antigenicity (epitope) is added as a tag sequence, it is possible to bind an antibody to the epitope. As the epitope, an HA (a peptide sequence of hemagglutinin of an influenza virus) tag, a myc tag, a FLAG tag, and the like can be exemplified. When the epitope tag is used, it is possible to easily purify the recombinant fibroin with a high specificity.

Furthermore, it is also possible to use recombinant fibroin from which a tag sequence is cut away using a specific protease. It is also possible to collect recombinant fibroin from which a tag sequence is cut away by carrying out a protease treatment on a protein adsorbed through the tag sequence.

More specific examples of the recombinant fibroin including the tag sequence include recombinant fibroin including (iii) an amino-acid sequence represented by a SEQ ID No. 7, a SEQ ID No. 9, or a SEQ ID No. 11 or including (iv) an amino-acid sequence having a sequence identity of 90% or more with an amino-acid sequence represented by the SEQ ID No. 7, the SEQ ID No. 9, or the SEQ ID No. 11.

The recombinant polypeptide may be recombinant fibroin including (iii) an amino-acid sequence represented by the SEQ ID No. 7, the SEQ ID No. 9, or the SEQ ID No. 11 or including (iv) an amino-acid sequence having a sequence identity of 90% or more with an amino-acid sequence represented by the SEQ ID No. 7, the SEQ ID No. 9, or the SEQ ID No. 11.

The amino-acid sequences represented by the SEQ ID No.s 6, 7, 8, 9, and 11 are respectively amino-acid sequences obtained by adding the amino-acid sequence represented by the SEQ ID No. 5 (including the His tag) to the N terminals of the amino-acid sequences represented by the SEQ ID No.s 1, 2, 3, 4, and 10. The recombinant fibroin (iv) is also a protein including the domain sequence represented by Formula 1: [(A)ₙ motif-REP]ₘ. The sequence identity is preferably 95% or more.

The structural protein preferably includes the recombinant polypeptide. When a molded article to be obtained includes the recombinant polypeptide as the structural protein, it is possible to adjust the bending elastic modulus, the bending strength, and the hardness of the molded article to desired numerical values.

### [Recombinant cell expressing structural protein]

A method for producing the recombinant polypeptide will be described below in detail. A target recombinant polypeptide can be produced by, for example, expressing the gene using a host transformed by an expression vector having a gene sequence that codes the structural protein and one or a plurality of regulatory sequences operably coupled to the gene sequence.

A method for producing the gene that codes the target recombinant polypeptide is not particularly limited. For example, the gene can be produced using a method in which a gene that codes a natural structural protein is used, amplified by a polymerase chain reaction (PCR) or the like, and cloned or a chemical synthesis. A chemical synthesis method of the gene is also not particularly limited, and it is possible to chemically synthesize a gene using a method in which oligonucleotides automatically synthesized using AKTA oligopilot plus 10/100 (manufactured by General Electric Company, Japan) or the like are coupled together by PCR or the like on the basis of the amino-acid sequence information of a structural protein procured from the web database of NCBI or the like. At this time, in order to facilitate the purification or confirmation of the protein, a gene that codes a polypeptide obtained by adding an amino-acid sequence made up of an initiation codon and a His 10 tag to the N terminal of the above-described amino-acid sequence may be synthesized.

The regulatory sequence is a sequence that suppresses the expression of a recombinant protein in the host (for example, a promoter, an enhancer, a ribosome-binding sequence, a transcription termination sequence, or the like) and can be appropriately selected depending on the kind of the host. As the promoter, an inducible promoter which functions in a host cell and is capable of inducing the expression of a target protein may be used. The inducible promoter is a promoter capable of controlling transcription using a physical factor such as the presence of an inducer (expression inducer), the absence of a repressor molecule, or an increase or decrease in the temperature, the osmotic pressure, or the pH value.

The kind of the expression vector can be appropriately selected depending on the kind of the host such as a plasmid vector, a virus vector, a cosmid vector, a fosmid vector, or an artificial chromosome vector. As the expression vector, an expression vector which is capable of autonomous replication in the host cell or capable of the integration of the host into a chrosome and contains a promotor at a location at which a gene that codes a target recombinant polypeptide can be transcribed is preferably used.

As the host, any of a prokaryote and a eukaryote such as an enzyme, a filamentous fungus, an insect cell, an animal cell, or a plant cell can be preferably used.

Preferred examples of the prokaryote include bacteria belonging to Escherichia, Brevibacillus, Serratia, Bacillus, Microbacterium, Brevibacterium, Corynebacterium, Pseudomonas, and the like.

Examples of the vector that introduces the gene that codes the target recombinant polypeptide include pBTrp2 (manufactured by Boehringer Ingelheim GmbH), pGEX (manufactured by Pharmacia Corporation), pUC18, pBluescriptII, pSupex, pET22b, pCold, pUB110, pNCO2 (refer to JP 2002-238569 A), and the like.

Examples of the host of the eukaryote include an enzyme and a filamentous fungus (mold or the like).

Examples of the enzyme include enzymes belonging to Saccharomyces, Pichia, Schizosaccharomyces, and the like. Examples of the filamentous fungus include filamentous fungi belonging to Aspergillus, Penicillium, Trichoderma, and the like.

Examples of the vector include YEp13 (ATCC37115), YEp24 (ATCC37051), and the like.

As a method for introducing the expression vector to the host cell, any method can be used as long as the method is to introduce DNA to the host cell. Examples thereof include a method in which a calcium ion is used [refer to Proc. Natl. Acad. Sci. USA, 69, 2110 (1972)], an electroporation method, a spheroplast method, a protoplast method, a lithium acetate method, a competent method, and the like.

As a method for expressing a gene using a host transformed by the expression vector, it is possible to carry out not only direct expression but also secretory production, fused protein expression, and the like according to a method described in Molecular Cloning Vol. 2 or the like.

The target recombinant polypeptide can be produced by, for example, culturing a host transformed by the expression vector according to the present invention in an incubation medium, preparing and accumulating the protein in the incubation medium, and picking the protein from the incubation medium. Regarding a method for culturing the host according to the present invention in the incubation medium, the host can be cultured according to an ordinarily-used method.

In a case in which the host according to the present invention is a prokaryote such as Bacillus coli or a eukaryote such as an enzyme, as the incubation medium of the host according to the present invention, any of a natural culture medium or a synthetic culture medium may be used as long as the culture medium contains a carbon source, a nitrogen source, an inorganic salt, and the like which enable the utilization of the host and enables the efficient culture of the host.

The carbon source needs to be a source enabling the utilization of the transformed microorganism, and it is possible to use, for example, glucose, fructose, sucrose, molasses containing the above-described substance, carbohydrates such as starch and starch hydrolysate, organic acids such as acetic acid and propionic acid, and alcohols such as ethanol and propanol.

As the nitrogen source, it is possible to use, for example, inorganic acids such as ammonia, ammonium chloride, ammonium sulfate, ammonium acetate, and ammonium phosphate, ammonium salts of an organic acid, other nitrogen-containing compounds, peptone, meat extracts, yeast extracts, corn steep liquor, casein hydrolysate, soybean cake, soybean cake hydrolysate, a variety of fermented fungus bodies, and digested substances thereof.

As the inorganic salt, it is possible to use, for example, potassium dihydrogenphosphate, dipotassium phosphate, magnesium phosphate, magnesium sulfate, sodium chloride, ferrous sulfate, manganese sulfate, copper sulfate, and calcium carbonate.

The prokaryote such as Bacillus coli or the eukaryote such as an enzyme can be cultured, for example, under the aerobic conditions of shaking culture or deep aeration stirring culture. The culture temperature is, for example, 15°C to 40°C. The culture time is generally 16 hours to 7 days. The pH of the incubation medium during culture is preferably maintained at 3.0 to 9.0. The pH of the incubation medium can be adjusted using an inorganic acid, an organic acid, an alkali solution, urea, calcium carbonate, ammonia, or the like.

In addition, during the culture, an antibiotic substance such as ampicillin or tetracycline may be added to the incubation medium if necessary. When a microorganism transformed using an expression vector for which an inducible promoter is used as a promoter is cultured, an inducer may be added to the culture medium if necessary. For example, when a microorganism transformed using an expression vector for which a lac promoter is used is cultured, isopropyl-P-D-thiogalactopyranoside or the like may be added to the culture medium, and, when a microorganism transformed using an expression vector for which a trp promoter is used is cultured, indoleacrylic acid may be added to the culture medium.

The recombinant polypeptide according to the present invention can be isolated and purified using a method that is ordinarily used to isolate and purify a protein. For example, in a case in which the recombinant polypeptide is expressed in a cell in a dissolved state, after the end of the culture, the host cell is collected by centrifugal separation, suspended in a water-based buffering solution, then, the host cell is crushed using an ultrasonic crusher, a French press, a Manton-Gaulin homogenizer, a DYNO MILL, or the like, and a cell-free extract is obtained. From the supernatant being obtained by centrifugally separating the cell-free extract, a purified preparation can be obtained using a method that is ordinarily used to isolate and purify a protein singly or the methods in combination. Examples of the above-described method include a solvent extraction method, a salting-out method using ammonium sulfate or the like, a desalination method, a precipitation method using an organic solvent, an anion-exchange chromatography method using a resin such as diethylaminoethyl SEPHAROSE (DEAE-SEPHAROSE) or DIAION HPA-75 (manufactured by Mitsubishi Kasei Kogyo Kabushiki Kaisha), a cation-exchange chromatography method using a resin such as S-Sepharose FF (manufactured by Pharmacia Corporation), a hydrophobic chromatography method using a resin such as BUTYL SEPHAROSE or PHENYL SEPHAROSE, a gel filtration method using a molecular sieve, an affinity chromatography method, a chromatofocusing method, an electrophoresis method such as isoelectric point electrophoresis, and the like.

In addition, in a case in which the recombinant polypeptide is expressed with forming an insoluble body in a cell, similarly, the host cell is collected, then, crushed, and centrifugally separated, thereby collecting an insoluble body of the recombinant polypeptide as a precipitation fraction. The collected insoluble body of the recombinant polypeptide can be solubilized using a protein denaturation agent. After the operation, a purified preparation of the recombinant polypeptide can be obtained using the same isolation and purification method as described above.

In a case in which the recombinant polypeptide is secreted to the outside of the cell, it is possible to collect the recombinant polypeptide from the culture supernatant. That is, the culture supernatant is acquired by treating a cultured substance using a method such as centrifugal separation, and a purified preparation can be obtained from the culture supernatant using the same isolation and purification method as described above.

### [Method for producing molded article]

A method for producing a molded article which is an embodiment includes a step 1 of adding water to a composition including a structural protein, a step 2 of introducing a water-containing composition obtained in the step 1 to a casting mold (mold) and molding the water-containing composition, and a step 3 of obtaining a molded article by drying the molded composition obtained in the step 2.

The step 1 is a step of adding water to a composition including a structural protein. The composition needs to comprise a structural protein. The composition may comprise only a structural protein or comprise a structural protein and an optional additive (for example, a plasticizer, a colorant, a filler, a synthetic resin, or the like). The content of the additive is preferably set to 50% by mass or less of the total amount of the structural protein. The composition typically has a shape of a powder shape (lyophilized powder or the like) or a fiber shape (a fiber being obtained by spinning or the like), and the molded article can be a fused body of a composition comprising a structural protein having the above-described shape.

The amount of water added in the step 1 is preferably 10 to 40 wt% and more preferably 20 to 30 wt% of the mass of the structural protein.

The step 2 is a step of introducing a water-containing composition obtained in the step 1 to a casting mold (mold) and molding the water-containing composition. In the molding, the composition may be heated and/or pressurized.

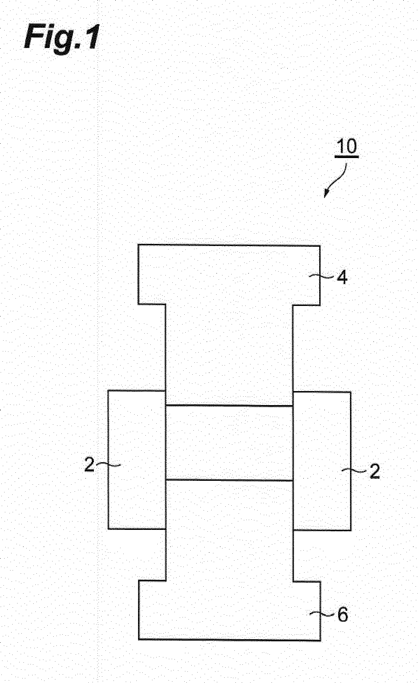

In the step 2, it is possible to mold the water-containing composition using, for example, a press molding machine. Fig. 1 is a schematic cross-sectional view of a press molding machine that can be used to produce the molded article. A press molding machine 10 illustrated in Fig. 1 includes a die 2 which has a through-hole formed therein and can be heated and an upper side pin 4 and a lower side pin 6 which are vertically movable in the through-hole of the die 2. The water-containing composition being obtained in the step 1 is introduced to a space generated by inserting the upper side pin 4 and the lower side pin 6 into the through-hole of the die 2, and the water-containing composition is compressed using the upper side pin 4 and the lower side pin 6 while heating the die 2, whereby the molded article can be obtained.

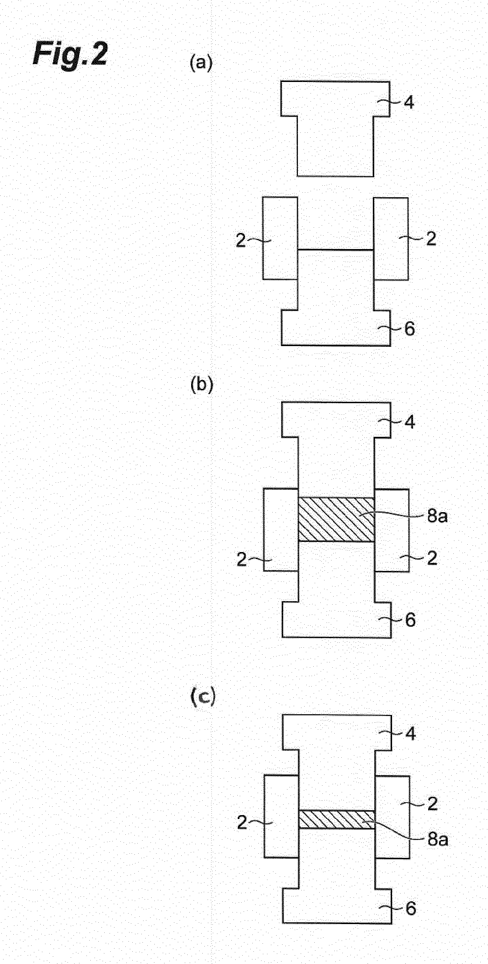

Fig. 2 illustrates step views of obtaining the molded article and schematic cross-sectional views of the press molding machine (a) before the introduction of the water-containing composition, (b) immediately after the introduction of the water-containing composition, and (c) with the water-containing composition in a state of being hot pressed. As illustrated in Fig. 2(a), the water-containing composition is introduced into the through-hole in a state in which only the lower side pin 6 is inserted into the through-hole of the die 2, but moisture is added thereto before the introduction or after the introduction, and then the water-containing composition is stirred. Subsequently, as illustrated in Fig. 2(b), the upper side pin 4 is inserted into the through-hole of the die 2 and lowered, and the die 2 begins to be heated, thereby hot pressing a water-containing composition 8a before hot pressing in the through-hole. The hot pressing is continued until the water-containing composition reaches a predetermined temperature in a state illustrated in Fig. 2(c) by lowering the upper side pin 4 until a previously-determined pressurization force is reached, thereby obtaining a water-containing composition 8b after hot pressing. After that, the temperature of the die 2 is lowered using a cooler (for example, a spot cooler), the upper side pin 4 or the lower side pin 6 is removed from the die 2 when the water-containing composition 8b reaches a predetermined temperature, and the content is removed. The pressurization may be carried out by lowering the upper side pin 4 in a state in which the lower side pin 6 is fixed, but may also be carried out by lowering the upper side pin 4 and the lifting the lower side pin 6 at the same time. The removed content is dried, thereby obtaining a molded article. The pressurization may be carried out by lowering the upper side pin 4 in a state in which the lower side pin 6 is fixed, but may also be carried out by lowering the upper side pin 4 and the lifting the lower side pin 6 at the same time.

The heating is carried out when the temperature of the die is preferably 80°C to 300°C, more preferably 100°C to 180°C, and still more preferably 100°C to 130°C. The pressurization is preferably carried out at a pressure of 20 MPa or more.

The step 3 is a step of drying the molded composition obtained in the step 2 to obtain the molded article. The molded composition is preferably dried by being left to stand in a vacuum and/or in an environment of 100°C for one minute to 10 days.

### Examples

Hereinafter, the present invention will be more specifically described on the basis of examples. However, the present invention is not limited to the following examples.

### (1) Production of structural protein expression line

The base sequence and the amino-acid sequence of fibroin derived from Nephila clavipes (GenBank accession number: P46804.1, GI: 1174415) were acquired from the web database of GenBank, the substitution, insertion, and deletion of amino-acid residues were carried out for the purpose of the improvement of the productivity, and furthermore, an amino-acid sequence (a tag sequence and a hinge sequence) represented by a SEQ ID No. 5 was added to an N terminal, thereby designing a recombinant fibroin having an amino-acid sequence represented by a SEQ ID No. 12 (hereinafter, also referred to as "PRT410").

Next, a gene that coded PRT410 was synthesized and entrusted. As a result, a gene in which an NdeI site was added immediately upstream of a 5' terminal of the gene and an EcoRI site was added immediately downstream of a 3' terminal was obtained. The gene was cloned to a cloning vector (pUC118), then, subjected to a restriction enzyme treatment using NdeI and EcoRI, and recombined into a protein expression vector pET-22b(+).

### (2) Expression of protein

Bacillus coli BLR (DE3) was transformed using a pET22b(+) expression vector including the gene that coded PRT410 obtained above. The transformed Bacillus coli was cultured in a 2 mL LB culture medium including ampicillin for 15 hours, and then the same culture fluid was added to a culture medium for seed cultivation (100 mL) shown in Table 1 so that OD₆₀₀ reached 0.005. The temperature of the culture fluid was maintained at 30°C, and the culture fluid was further cultured in a flask for 15 hours so that OD₆₀₀ reached 5, thereby obtaining a seed culture fluid.

**[Table 1]**

| Chemical | Concentration |
|---|---|
| Glucose | 5.0 g/L |
| KH₂PO₄ | 4.0 g/L |
| K₂HPO₄ | 9.3 g/L |
| Yeast Extract | 6.0 g/L |
| Ampicillin | 0.1 g/L |

The obtained seed culture fluid was added to a jar fermenter to which a production culture medium (500 mL) shown in Table 2 had been added so that OD₆₀₀ reached 0.05. The temperature of the culture fluid was maintained at 37°C, the pH was controlled so as to become constant at 6.9, and the culture fluid was cultured with the concentration of dissolved oxygen in the culture fluid set to be maintained at 20% of the saturation concentration of dissolved oxygen. Meanwhile, as a defoamer, ADEKA NOL LG-295S (manufactured by ADEKA Corporation) was used.

**[Table 2]**

| Chemical | Concentration |
|---|---|
| Glucose | 12.0 g/L |
| KH₂PO₄ | 9.0 g/L |
| MgSO₄·7H₂O | 2.4 g/L |
| Yeast Extract | 15 g/L |
| FeSO₄·7H₂O | 0.04 g/L |
| MnSO₄·5H₂O | 0.04 g/L |
| CaCl₂·2H₂O | 0.04 g/L |
| LG-295S | 0.1 mL/L |

Immediately after the complete consumption of glucose in the production culture medium, a feed fluid (455 g/L of glucose and 120 g/L of yeast extract) was added at a rate of 1 mL/minute. The temperature of the culture fluid was maintained at 37°C, the pH was controlled to be constant at 6.9, and the culture fluid was cultured. In addition, the concentration of dissolved oxygen in the culture fluid was set to be maintained at 20% of the saturation concentration of dissolved oxygen, and the culture fluid was cultured for 20 hours. After that, a 1 M isopropyl-β-thiogalactopyranoside (IPTG) aqueous solution was added to the culture fluid so that the final concentration reached 1 mM, and the target protein was expressed and induced. At a point in time when 20 hours elapsed from the addition of IPTG, the culture fluid was centrifugally separated, and solid was collected. SDS-PAGE was carried out using fungus bodies prepared from the culture fluid before the addition of IPTG and after the addition of IPTG, and the expression of the target protein was confirmed from the appearance of a band of the target protein size which was dependent on the addition of IPTG.

### (3) Purification of structural protein

The fungus body collected after two hours from the addition of IPTG was washed with a 20 mM Tris-HCl buffer solution (pH: 7.4). The washed fungus body was suspended in a 20 mM Tris-HCl buffer solution (pH: 7.4) including phenylmethylsulfonyl fluoride (PMSF) (approximately 1 mM), and cells were broken using a high-pressure homogenizer (GEA Niro Soavi SpA). The broken cells were centrifugally separated, thereby obtaining a precipitate. The obtained precipitate was washed with a 20 mM Tris-HCl buffer solution (pH: 7.4) until the precipitate became highly pure. The washed precipitate was suspended in an 8 M guanidine buffer solution (8 M guanidinium hydrochloride, 10 mM sodium dihydrogen phosphate, 20 mM NaCl, 1 mM Tris-HCl, pH: 7.0) so as to be a concentration of 100 mg/mL, stirred using a stirrer at 60°C for 30 minutes, and dissolved. After the dissolution, dialysis was carried out using a dialysis tube (cellulose tube 36/32 manufactured by Sanko Junyaku Co., Ltd.) and water. A white aggregate protein obtained after the dialysis was collected by centrifugal separation, moisture was removed using a freeze dryer, and lyophilized powder was collected.

### <Production of molded article>

### (Example 1)

A sample obtained by adding pure water (30 wt%) to the lyophilized powder (1.35 g) was introduced to a through-hole of a die 2 (the die 2 was a cylindrical die and had a circular through-hole having a diameter of 20 mm) of a press molding machine 10 illustrated in Fig. 1. After the entire sample was introduced to the through-hole, the die 2 began to be heated, and the sample was pressurized by inserting an upper side pin 4 and a lower side pin 6 into the through-hole using a hot press machine. At this time, the pressurization condition of the sample was controlled so that the pressure reached 31 MPa. The heating was stopped when the temperature of the sample reached 170°C, the sample was cooled, then, removed from the die, then, injected into a vacuum heating furnace, and dried at 100°C for 16 hours in a vacuum, thereby obtaining a molded article having a disc shape having a diameter of 20 mm and a thickness of 2 mm.

### (Comparative Example 1)

A molded article was obtained by hot pressing the above-described lyophilized powder in the same manner as in Example 1 except for the fact that the amount of the lyophilized powder was changed to 0.8 g in order to match the thickness of a compact to be obtained, and the achieving temperature during the hot pressing was set to 170°C in order to control the temperature increase during molding. In Comparative Example 1, drying was not carried out after hot pressing.

### <Production of test specimen>

The surface of the molded article was polished with sheets of polishing paper #600 and #1000 (based on JIS R6001: 1988 the grain size distribution of fine powder for precision polishing), flattened, and worked (cut) to a 4 mm-wide plate shape including a disc-shaped central line, thereby obtaining an approximately 20 mm×4 mm×2 mm test specimen.

### <Measurement of bending elastic modulus and bending strength>

On the obtained test specimen, a three-point bending test was carried out in AUTOGRAPH (manufactured by Shimadzu Corporation, trade name: AGS-X). At this time, the distance between supporting pins for three-point bending was fixed to 14 mm, and the measurement rate was set to 1 mm/minute. In addition, the dimensions of the test specimen were measured using a micrometer, then, the test specimen was installed on the supporting pins, and the bending elastic modulus and the bending strength were measured.

### <Measurement of Micro Vickers hardness>

The Vickers hardness of the obtained test specimen was measured using a Micro Vickers hardness tester (manufactured by Shimadzu Corporation, trade name: HMV-G).

### <Measurement of moisture percentage>

The moisture percentage of the test specimen during the measurement of the bending strength was measured using a Karl Fischer-type moisture meter (manufactured by Metrohm AG, trade name: 860 KF Thermoprep, 851 Titrando, 801 Stirrer). At this time, the test specimen was heated to 180°C.

The respective measurement results are shown in Table 3. The molded articles of Example 1 and Comparative Example 1 had almost the same degree of moisture percentages. Example 1 improved more significantly than Comparative Example 1 in both of the bending elastic modulus and the bending strength.

**[Table 3]**

| | Addition of moisture | Drying time | Moisture percentage | Vickers hardness |
|---|---|---|---|---|
| Example 1 | 30 wt% | 16 h | 2.7% | 45 HV |
| Comparative Example 1 | None | None | 2.4% | 40 HV |

| | Three-point bending strength | Three-point bending elastic modulus | | |
|---|---|---|---|---|
| Example 1 | 141 MPa | 7.3 GPa | | |
| Comparative Example 1 | 98 MPa | 6.8 GPa | | |

### Reference Signs List

2: DIE, 4: UPPER SIDE PIN, 6: LOWER SIDE PIN, 8a: WATER-CONTAINING COMPOSITION BEFORE HOT PRESSING, 8b: WATER-CONTAINING COMPOSITION AFTER HOT PRESSING, 10: PRESS MOLDING MACHINE

## Claims

1. A method for producing a molded article, comprising:
a step 1 of adding water to a composition including a structural protein;
a step 2 of molding a water-containing composition obtained in the step 1; and
a step 3 of drying the molded composition obtained in the step 2 to obtain the molded article.

2. The method according to Claim 1, wherein the step 2 includes hot pressing.

3. The method according to Claim 1 or 2, wherein the structural protein includes a spider silk protein or a recombinant polypeptide thereof.

4. A molded article which is obtained by using the method according to any one of Claims 1 to 3.

5. A molded article which is a molded article of a composition including a structural protein and has a bending elastic modulus of more than 6.9 GPa.

6. The molded article according to Claim 5 which is a hot press-molded article.

7. The molded article according to Claim 5 or 6, wherein the structural protein includes a spider silk protein or a recombinant polypeptide thereof.
